# EUROPÄISCHE PATENTSCHRIFT

(11) **EP 1 989 986 B1**
(45) Veröffentlichungstag und Bekanntmachung des Hinweises auf die Patenterteilung: **16.09.2009**
(21) Anmeldenummer: 08004752.5
(22) Anmeldetag: 14.03.2008
(51) Int. Cl.: A47L 15/00, A47L 15/42, A47L 15/46, A61G 9/02, A61L 2/24, A61L 2/26

(54) **Desinfektionssteuerung durch Zielerregerauswahl**
Disinfection control by selecting target germs
Commande de désinfection par la sélection d'agents pathogènes cibles

(30) Priorität: 07.05.2007 DE 102007021245
(43) Veröffentlichungstag der Anmeldung: 12.11.2008
(73) Patentinhaber: MEIKO Maschinenbau GmbH & Co. KG, 77652 Offenburg (DE)
(72) Erfinder: Scheringer, Stefan, Dr., 77654 Offenburg (DE); Peukert, Thomas, Dr., 77704 Oberkirch (DE); Braun, Markus, 77656 Offenburg (DE)
(74) Vertreter: Hörschler, Wolfram Johannes

(56) Entgegenhaltungen:
- WO-A-20/06053634
- DE-A1- 19 509 877

## Beschreibung

### Gebiet der Erfindung

Die Erfindung betrifft ein Verfahren zur Steuerung eines Reinigungsgerätes sowie ein Reinigungssystem, welches insbesondere zur Durchführung des vorgeschlagenen Verfahrens geeignet sein soll. Das vorgeschlagene Verfahren und das vorgeschlagene Reinigungssystem sind einsetzbar zur Reinigung einer Vielzahl von Objekten. Ein besonderer Schwerpunkt der vorliegenden Anmeldung liegt auf der Reinigung von Pflegegeräten und Krankenhausgeräten, welche mit einer hohen Menge an Flüssigkeitsabfall bzw. anderen Arten von Abfällen (z.B. Ausscheidungen, Verschmutzungen, Salbenresten etc.) kontaminiert sind, insbesondere Steckbeckenspülern zur Reinigung von Bettpfannen, Steckbecken, Urinflaschen oder ähnlichem.

### Stand der Technik

Aus vielen Bereichen des täglichen Lebens, der Technik, der Naturwissenschaften und der Medizin bzw. dem Pflegebereich sind Reinigungssysteme für eine Vielzahl von zu reinigenden Objekten bekannt. Als Beispiele, welche das mögliche Anwendungsspektrum der vorliegenden Erfindung jedoch nicht beschränken, können Geschirrspülmaschinen genannt werden, welche beispielsweise im gewerblichen Bereich als Einkammer- oder Mehrkammergeschirrspülmaschinen in Benutzung sind. Als weiteres Beispiel seien Reinigungsvorrichtungen für medizinische Geräte genannt, bei welchen hohe Flüssigkeitsmengen als Abfall anfallen, beispielsweise Steckbeckenspüler für die Reinigung von Steckbecken, Bettpfannen, Urinflaschen, Nachtgeschirr oder ähnlichen Behältnissen aus dem medizinischen Bereich oder Pflegebereich. Hier und im Folgenden sind unter "Flüssigkeitsabfällen" dementsprechend auch Mengen an anderen Abfällen zu verstehen, wie beispielsweise menschlichen oder tierischen Ausscheidungen, Salbenresten oder ähnlichen Abfällen.

Viele Reinigungsvorrichtungen dienen der Reinigung von Objekten, welche unmittelbar oder mittelbar mit dem menschlichen Körper in Verbindung kommen, welche somit als Überträger von Krankheiten, insbesondere Infektionen, dienen können und bei denen somit ein besonderer Wert auf eine ausreichende Desinfektion bzw. eine Erzielung eines bekannten Hygienegrades gelegt wird.

Die Messung und Sicherstellung eines Hygienegrades bzw. einer Keimreduzierung sind Gegenstand zahlreicher bekannter Verfahren und Normen. So ist insbesondere der Zusammenhang von Temperatur und Zeit auf eine Keimreduzierung die Grundlage von Vorschriften und Normen, welche die Reinigungswirkung in Spülmaschinen sicherstellen sollen. Aufgrund von Versuchen, die an Mehrtankspülmaschinen durchgeführt wurden, mit dem Ziel, die Prozessparameter festzulegen, bei denen eine sichere Hygienisierung des Spülgutes erreicht wird, wurde mit der DIN 10510 C3 für Deutschland ein Verfahren entwickelt, das eine Empfehlung hinsichtlich Temperatur, Reinigerkonzentration und Dauer zwischen dem ersten Kontakt des zu reinigenden Gutes mit der Spülflüssigkeit der ersten Spülzone bis zum Verlassen der Klarspülzone gibt. Mit diesen empfohlenen Parametern wird dann die Mehrkammerspülmaschine in den einzelnen Verfahrenszonen betrieben, um im Betrieb die geforderte Keimreduktion zu erreichen. Grundlage der genannten Norm ist die Keimreduktion von definiert angeschmutzten Prüfkörpern nach dem Reinigungsprozess über so genannte Abklatschuntersuchungen. Als Testkeim bzw. als Testorganismus wird bei diesem Test E. faecium ATCC 6057 verwendet.

In den USA wird der Zusammenhang von Temperatur und Zeit auf die Keimreduzierung durch das NSF3-Standard-Verfahren beschrieben. Basis dieser Vorgabe ist die aus Versuchen ermittelte Keimreduktion von Tuberkulosebakterien durch die Einwirkung einer Temperatur über die Zeit. Das Einwirken der Temperatur über die Zeit wird dabei als "Wärmeäquivalent" bezeichnet. Wie viele Wärmeäquivalente pro Sekunde bei welcher Temperatur erzielt werden, ist in einer Tabelle in diesem Verfahren niedergeschrieben. Anhand dieser Tabelle werden für Spülmaschinen eine Mindesttemperatur für das Spülwasser der Spülzone sowie der Nachspülzone definiert, welche die Spülmaschine erreichen muss, um die gemäß dieser Norm geforderte Keimreduktion zu erreichen.

Aus US 6,615,850 B1 ist ein Verfahren zum Betrieb einer Geschirrspülmaschine bekannt, welches eine kontinuierliche Aufnahme der Nachspülwassertemperatur innerhalb der Geschirrspülmaschine sowie ein Aufrechterhalten einer optimalen Temperatur umfasst. Es wird ein kumuliertes Wärmeäquivalent (Heat Unit Equivalent, H.U.E.) aufgenommen, und der Nachspülzyklus wird abgebrochen, sobald ein vorgegebener H.U.E.-Wert erreicht ist.

In DE 195 09 877 A1 wird eine Vorrichtung zum Reinigen und Desinfizieren von Steckbecken, Urinflaschen und dergleichen beschrieben. Die Desinfektion erfolgt dabei durch Dampf. Unter anderem wird ausgeführt, dass mittels einer elektronischen Regeleinrichtung die Dauer der Desinfektion der Steckbecken sowie der Urinflaschen in der Reinigungskammer exakt nach Wunsch gesteuert werden kann, um, in Abhängigkeit von der Absterbegeschwindigkeit der jeweils relevanten Keime, nicht nur eine genaue, sondern auch eine äußerst wirtschaftliche Desinfektion selbst äußerst resistenter Keime zu ermöglichen.

In WO 2006/053634 A1 wird ein Verfahren zur Beurteilung und Sicherstellung der thermischen Hygienewirkung auf Spülgut in einer Spülkammer einer Geschirrspülmaschine offenbart. Über einen Temperatursensor und eine Steuerung kann dabei die thermische Hygienewirkung erfasst werden.

Gemäß dem Stand der Technik wird also bei üblichen Spülmaschinen und anderen Reinigungsvorrichtungen durch das Spülpersonal ein entsprechend vorkonfiguriertes Spülprogramm gewählt. Beispielsweise betätigt ein Bediener entsprechende Tasten und/oder Schalter. Die Erreichung des Reinigungsergebnisses und/oder einer avisierten Keimreduzierung wird durch entsprechende Parametrisierung für ein sehr breites Einsatzspektrum unterstellt und gegebenenfalls in Typprüfungen und/oder Validierungen bestätigt.

Nachteilig an den bekannten Reinigungsvorrichtungen und Reinigungsverfahren ist es jedoch, dass diese in der Regel unspezifisch hinsichtlich der tatsächlich vorliegenden Keime oder Krankheitserreger funktionieren. So wird beispielsweise das Spülgut mit einem fest vorgegebenen Thermoäquivalent beaufschlagt, welches beispielsweise hoch genug gewählt werden kann, um gängige Krankheitserreger oder Keime weitgehend abzutöten. In der Regel wird dabei ein "Overkill" betrieben, da die veranschlagten Thermoäquivalente in der Regel hoch genug angesetzt werden, um alle gängigen Arten von Keimen abzutöten. Dies ist jedoch, insbesondere im Bereich des gewerblichen Einsatzes mit hohen Spülgut-Durchsätzen mit einer starken Umweltbelastung und einem erhöhten Energieverbrauch verbunden, wodurch die Betriebskosten und die Umweltverträglichkeit derartiger Reinigungsvorrichtungen in vielen Fällen stark zu wünschen übrig lassen. Wird hingegen der veranschlagte Thermoäquivalent-Wert zu niedrig angesetzt, so besteht die Gefahr, dass viele Keime nicht abgetötet werden und das Spülgut auf diese Weise zu einem Überträger von Infektionen werden kann.

### Aufgabe der Erfindung

Aufgabe der vorliegenden Erfindung ist es daher, ein Verfahren zum Betrieb eines Reinigungsgerätes vorzuschlagen, welches einerseits eine zuverlässige Reinigungs- und Desinfektionswirkung bereitstellt und so das Risiko einer Verbreitung von Infektionen vermindert, welches jedoch andererseits umweltfreundlich ist und unter verringertem Energiebedarf implementierbar ist.

### Beschreibung_der Erfindung

Diese Aufgabe wird durch ein Verfahren gemäß dem Anspruch 1 und ein Reinigungssystem gemäß Anspruch 12 gelöst. Vorteilhafte Weiterbildungen der Erfindung, welche einzeln oder in Kombination realisiert werden können, sind in den abhängigen Ansprüchen aufgeführt.

Ein Grundgedanke der vorliegenden Erfindung besteht darin, die Reinigung gezielt auf einen oder mehrere Typen von bekannten Zielkeimen abzustimmen. Dabei wird die Tatsache berücksichtigt, dass in vielen Fällen Informationen vorliegen, mit welcher Art von Keimen das Reinigungsgut (im Folgenden auch "Objekt" oder "Spülgut" genannt) kontaminiert ist. Derartige Informationen können aus verschiedenen Quellen stammen, beispielsweise in einem Krankenhaus aus einer vorangehenden Diagnose, mit welchem Krankheitserreger ein bestimmter Patient, der das Objekt zuvor genutzt hat, infiziert ist. Andere derartiger Informationsquellen schließen regionale, nationale oder globale Ausbreitungen von Infektionen ein, welche zum Beispiel vom Gesundheitsministerium oder von Ärztevereinigungen bereitgestellt werden können. Auch bei solchen Warnungen kann entsprechend reagiert werden.

Diese Information über den bzw. die Zielkeime kann nunmehr genutzt werden, um den Reinigungsprozess und/oder den Desinfektionsprozess gezielt auf den bzw. die Zielkeime anzupassen. So kann, ohne einen "Overkill" zu betreiben, welcher weder betriebswirtschaftlich noch medizinisch sinnvoll wäre, der Prozess (beispielsweise unter Ansetzung einer Mindest-Reinigungswirkung) derart ausgestaltet werden, dass mit hoher Wahrscheinlichkeit alle Zielkeime abgetötet werden.

Das vorgeschlagene Verfahren kann auf den Betrieb von Reinigungsgeräten der verschiedensten Arten angewandt werden, beispielsweise der eingangs bei der Beschreibung des Standes der Technik erwähnten Arten. Ein besonderen Schwerpunkt des vorgeschlagenen Verfahrens liegt auf der Reinigung von Geschirr sowie auf der Reinigung von Pflegegeräten der eingangs erwähnten Art. Das Reinigungsgerät soll eingerichtet sein, um einen Reinigungsprozess und/oder einen Desinfektionsprozess eines Objektes mit keimtötender Wirkung durchzuführen.

Das Verfahren weist die im Folgenden genannten Schritte auf, wobei die dargestellte Reihenfolge bevorzugt, jedoch nicht zwingend ist und wobei einzelne Schritte beispielsweise auch zeitlich parallel, wiederholt oder zeitlich überlappend durchgeführt werden können. Weiterhin können auch zusätzliche, nicht erwähnte Verfahrensschritte durchgeführt werden.

Zunächst wird eine Information über einen Zielkeim von dem Reinigungssystem empfangen, mit welchem das Objekt kontaminiert sein könnte. Diese Information kann beispielsweise aus einer der oben beschriebenen Informationsquellen stammen und wird vorzugsweise von einem Benutzer manuell in das Reinigungsgerät eingegeben. Auch andere Arten des Informationsempfangs sind jedoch denkbar, beispielsweise ein Einlesen der Informationen über eine Schnittstelle zu einem Computer oder Computernetzwerk. Weitere Einzelheiten werden unten im Zusammenhang mit dem Reinigungssystem erläutert.

Entsprechend dem Zielkeim wird anschließend mindestens ein Prozessparameter des Reinigungsprozesses von dem Reinigungssystem derart ausgewählt, dass der Zielkeim bei dem Reinigungsprozess mit hoher Wahrscheinlichkeit abgetötet wird. Die Wahl des Prozessparameters erfolgt durch das Reinigungssystem. Bei dem Prozessparameter kann es sich beispielsweise um eine Temperatur, eine Einwirkzeit eines Reinigungsschrittes, eine Einwirkzeit eines thermischen Behandlungsschrittes, eine Auswahl eines Desinfektionsmittels oder eine Menge eines Desinfektionsmittels handeln. Auch andere Prozessparameter, welche Einfluss auf die Reinigungs- bzw. Desinfektionswirkung haben, sind jedoch einsetzbar, sowie Kombinationen der genannten oder anderer Prozessparameter.

Anschließend wird der Reinigungsprozess mit dem Prozessparameter bzw. dem Prozessparametersatz, welcher entsprechend dem bzw. den Zielkeimen ausgewählt wurde, durchgeführt. Weist der Reinigungsprozess selbst mehrere Prozessschritte auf, so können die vorangehenden Schritte, insbesondere der Schritt der Auswahl eines dem Zielkeim entsprechenden Parametersatzes, auch für jeden Prozessschritt einzeln durchgeführt werden, beispielsweise für einen Umwälzspülvorgang und einen anschließenden Nachspülvorgang separat.

Die Information über den Zielkeim wird zunachst genutzt, um einen Erfahrungswert eines dem Zielkeim entsprechenden Zielthermoäquivalents auszuwählen. Dieses Zielthermoäquivalent kann beispielsweise theoretisch oder auch empirisch durch Keimabtötungsversuche verschiedener Keime ermittelt werden und stellt das Thermoäquivalent dar, bei welchem der Zielkeim mit hoher Wahrscheinlichkeit abgetötet ist. Beispielsweise kann dafür ein Mindestprozentsatz abgetöteter Zielkeime vorgegeben werden. Der Reinigungsprozess wird dann derart durchgeführt, dass das Objekt mindestens mit dem Zielthermoäquivalent beaufschlagt wird.

Dabei ist es wiederum bevorzugt, die aus den verschiedenen Standards bekannten Thermoäquivalente einzusetzen. Beispielsweise können wiederum Thermoäquivalente gemäß dem EN ISO 15883-Standard oder dem NSF3-Standard verwendet werden, insbesondere A₀-Werte und/oder H.U.E.-Werte. Auch andere Definitionen von Thermoäquivalenten können eingesetzt werden, welche grundsätzlich auf einer Beaufschlagung von zu reinigendem Gut mit thermischer Energie über eine bestimmte Zeit hinweg beruhen.

Beispielsweise kann der Reinigungsprozess dann derart durchgeführt werden, dass in einer oder mehreren Behandlungszonen einer Reinigungsvorrichtung eine Temperatur gemessen bzw. überwacht wird, mit welcher das Objekt beaufschlagt wird. Weiterhin kann eine Einwirkzeit, in welcher das Objekt mit der Temperatur beaufschlagt wird, gemessen werden, um so Thermoäquivalente kumuliert berechnen zu können. Dabei können die verschiedenen, teilweise aus dem Stand der Technik bekannten Verfahren eingesetzt werden, wie beispielsweise das eingangs beschriebene Verfahren gemäß US 6,615,850 B1, bei welchem die Temperatur beim Nachspülen in der Spülkammer überwacht und die Thermoäquivalente gezählt werden.

Um eine Zuordnung eines oder mehrerer Zielkeime zu einem oder mehreren Prozessparametern vornehmen zu können, mit oder ohne Umrechnung in einen Zielthermoäquivalenzwert, können verschiedene, dem Fachmann bekannte Techniken eingesetzt werden. Beispielsweise können entsprechende Datenbanken, Listen, Tabellen, Matrizen, Korrelationskurven oder andere Zuordnungen verwendet werden, welche ein- oder mehrdimensional sein können und welche entsprechende Zuordnungen enthalten. Dabei können auch mehrere Zielkeime zu Zielkeimgruppen oder Zielkeimklassen mit gemeinsamen oder ähnlichen Parametern zusammengefasst sein. Zur Sicherstellung einer Mindest-Desinfektionswirkung kann, unabhängig vom eingegebenen Zielerreger, ein Mindestparametersatz vorgegeben werden, welcher beispielsweise eingesetzt werden kann, wenn kein Zielerreger bekannt und/oder eingelesen ist bzw. wird.

Neben dem beschriebenen Verfahren in einer der dargestellten Ausführungsformen wird weiterhin ein Reinigungssystem zur Reinigung eines Objektes vorgeschlagen, welches vorzugsweise eingerichtet ist, um das beschriebene Verfahren in einer der dargestellten Ausführungsformen umzusetzen bzw. zu verwenden. Das Reinigungssystem umfasst mindestens eine Reinigungsvorrichtung, welche eingerichtet ist, um einen Reinigungsprozess des Objekts mit keimtötender Wirkung durchzuführen.

Zum Empfangen der Information über den bzw. die Zielkeime kann das Reinigungssystem insbesondere eine Eingabevorrichtung umfassen, die einem Benutzer die Eingaben eines Zielkeims ermöglicht. Beispielsweise kann einem Benutzer eine Auswahl bekannter Zielkeime vorgegeben werden, für welche entsprechende Parametersätze vorliegen. Sollte keiner dieser Zielkeime der Vorgabe des Benutzers entsprechen, so kann beispielsweise ein "Default"-Wert vorgegeben werden, beispielsweise der oben beschriebene Mindestparametersatz. Auch mehrere Mindestparametersätze sind möglich. Die Eingabevorrichtung kann beispielsweise ein oder mehrere Displays (zum Beispiel einen Touch-Screen), eine Tastatur, eine Maus, einen Trackball oder ähnliche, dem Fachmann bekannte Eingabevorrichtungen umfassen. Insbesondere kann die Eingabevorrichtung auch einen Computer mit entsprechenden Ein- und Ausgabemitteln umfassen, beispielsweise einen Personalcomputer oder einen kompakten, anwendungsspezifischen Prozesssteuerungs-Computer. Auch entsprechende Datenspeicher und andere, üblicherweise in Computersystemen angeordnete Elemente können umfasst sein. Insbesondere kann die Eingabevorrichtung auch eine drahtlose Schnittstelle umfassen, beispielsweise eine Infrarot- oder Bluetooth-Schnittstelle.

Alternativ oder zusätzlich zu der vorgeschlagenen Eingabevorrichtung kann das Reinigungssystem auch eine Schnittstelle umfassen, um Informationen zu empfangen. Beispielsweise können dabei eine oder mehrere der oben genannten Informationsquellen über die Zielkeime genutzt werden. Zu diesem Zweck kann beispielsweise das Reinigungssystem an ein entsprechendes Netzwerk angeschlossen sein. Dabei kann das Reinigungssystem auch eine Mehrzahl von Reinigungsvorrichtungen umfassen, welche beispielsweise über eine zentrale Steuerung gesteuert werden können. Beispielsweise kann diese zentrale Steuerung eingerichtet sein, um zentral für alle oder mehrere der Reinigungsvorrichtungen die Information über den Zielkeim zu empfangen.

Eine derartige Weiterbildung der Erfindung macht sich insbesondere in Krankenhäusern oder Pflegeheimen sowie in anderen Einrichtungen mit zahlreichen Reinigungsvorrichtungen, welche vorzugsweise synchron auf einen bestimmten Zielkeim angepasst werder sollen, positiv bemerkbar. Beispielsweise kann die zentrale Steuerung in einer zentralen Technikwarte eines Krankenhauses oder Pflegeheims angeordnet sein und kann beispielsweise über ein Datenübertragungsnetzwerk, wie beispielsweise das Internet, mit Informationen über zu erwartende Zielkeime versorgt werden. Alternativ oder zusätzlich können auch Patientendatenbanken mit dieser zentralen Steuerung verbunden sein, welche beispielsweise Information liefern können, dass sich auf bestimmte Stationen Patienten mit einem bestimmten Krankheitsbild bzw. mit bestimmten Infektionen befinden.

Entsprechend kann die zentrale Steuerung dann beispielsweise Reinigungsvorrichtungen, welche bekanntermaßen Objekte reinigen, die mit den entsprechenden Patienten direkt oder indirekt in Berührung kamen, steuern, um die gewünschte Desinfektion- bzw. Reinigungswirkung für die zu erwartenden Zielkeime zu erzielen. Die zentrale Steuerung kann wiederum einen oder mehrere Computer oder Computernetzwerke umfassen, welche beispielsweise wiederum mit entsprechenden Ein- und Ausgabemitteln, Datenspeichern und/oder Schnittstellen ausgestattet sein können. Insbesondere können die Steuerungen programmtechnisch eingerichtet sein, um ein Verfahren gemäß einer der oben beschriebenen Ausführungsvarianten auf einem oder mehreren der angeschlossenen Reinigungsvorrichtungen des Reinigungssystems zu steuern oder durchzuführen.

Wird eine derartige zentrale Steuerung für mehrere Reinigungsvorrichtungen verwendet, so macht sich eine der oben beschriebenen Verfahrensvarianten besonders positiv bemerkbar, nämlich die Verfahrensvariante der Zuordnung von Zielthermoäquivalenten. Auf diese Weise können beispielsweise die unterschiedlichsten Arten von Reinigungsvorrichtungen zentral gesteuert werden, ohne dass unmittelbar zentral Einfluss auf die vorrichtungsspezifischen Prozessparameter genommen werden muss.

Die Zuordnung eines Zielthermoäquivalenz zu dem vorgegeben Zielkeim stellt die Umrechnung in eine gemeinsame "Währung" dar, mit welcher viele Reinigungsvorrichtungen, unabhängig von deren genauer Funktion, arbeiten können. So kann in der zentralen Steuerung zunächst dem Zielkeim ein Zielthermoäquivalent zugeordnet werden. Beispielsweise kann hierbei wiederum eine Liste oder eine andere Zuordnung der oben beschriebenen Art verwendet werden. Dieses zugeordnete Zielthermoäquivalent kann dann an die einzelnen Reinigungsvorrichtungen weitergeleitet werden. Erst dort wird dann, beispielsweise mittels entsprechender Steuerungen, entsprechend dieser Zielthermoäquivalent-Vorgabe ein Reinigungsprozess derart durchgeführt werden, dass die zu reinigenden Objekte mit dem Zielthermoäquivalent beaufschlagt werden. Auf diese Weise wird zum einen eine Umprogrammierung jeder einzelnen Reinigungsvorrichtung vermieden, gleichzeitig jedoch der Rechenaufwand bzw. die erforderlichen Ressourcen für die Einstellung der optimalen Prozessparameter möglichst günstig verteilt.

Neben den oben beschriebenen Geschirrspülmaschinen, insbesondere gewerblichen Geschirrspülmaschinen, welche als Einkammer- oder Durchlaufgeschirrspülmaschinen ausgestaltet sein können, sowie den ebenfalls erwähnten Reinigungsvorrichtungen (auch als Reinigungs- und Desinfektionsgeräte bezeichnet) für die Reinigung von medizinischen Geräten oder Pflegeutensilien mit hohem Flüssigkeitsanfall, insbesondere Steckbeckenspülern für die Reinigung von Steckbecken, Urinflaschen, Bettpfannen, Nachtgeschirr etc. ist das vorgeschlagene Reinigungssystem und das vorgeschlagene Verfahren auch für ein Vielzahl anderer Arten von Objekten bzw. Arten von Spülgut einsetzbar. Insbesondere sind hier Reinigungsvorrichtungen für die Desinfektion von medizinischen Geräten ohne oder mit geringem Flüssigkeitsanfall zu nennen, welche beispielsweise für chirurgisches Besteck oder für Dentalbesteck eingesetzt werden können. Insbesondere können diese wiederum thermische Desinfektions- oder Sterilisationsvorrichtungen (z.B. einen Autoklaven) und/oder Dampfdesinfektionsvorrichtungen umfassen. Auch andere Arten von Objekten sind jedoch reinigbar.

Weitere Einzelheiten und Merkmale der Erfindung ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: eine schematische Darstellung eines erfindungsgemäßen Reinigungssystems; und
- Figur 2: einen Ablaufplan eines Ausführungsbeispiels eines erfindungsgemäßen Verfahrens zum Betrieb des Reinigungssystems gemäß Figur 1.

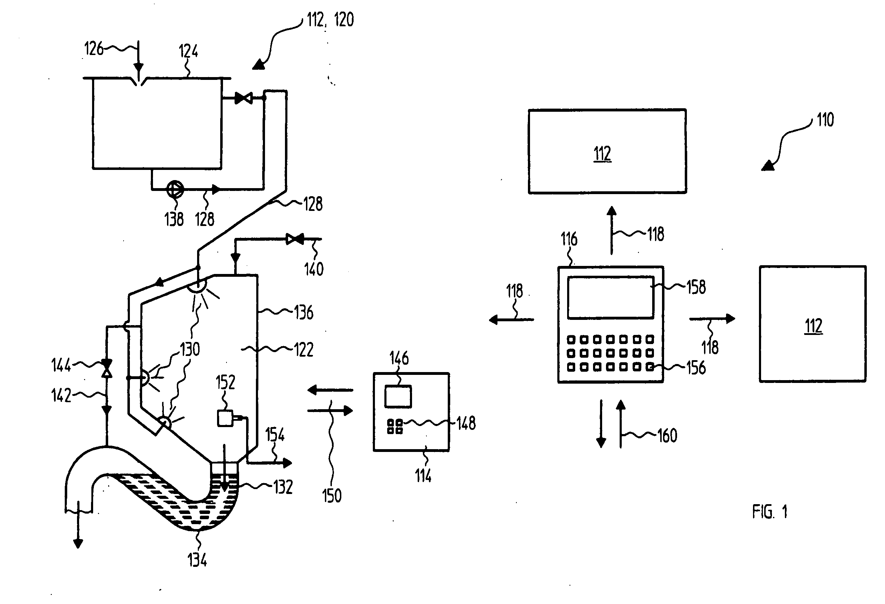

In Figur 1 ist ein Ausführungsbeispiel eines erfindungsgemäßen Reinigungssystems 110 dargestellt. Das Reinigungssystem 110 umfasst in diesem Ausführungsbeispiel mehrere Reinigungsvorrichtungen 112, welche in Figur 1 zum Teil lediglich schematisch dargestellt sind. Die Reinigungsvorrichtungen 112 können insbesondere jeweils eine eigene Steuerung 114 umfassen.

Weiterhin weist das Reinigungssystem 110 eine zentrale Steuerung 116 auf, welche beispielsweise mit den Steuerungen 114 der einzelnen Reinigungsvorrichtungen kommunizieren kann. Diese Kommunikation, welche ein- oder bidirektional sein kann, kann insbesondere über eine in Figur 1 mit der Bezugsziffer 118 bezeichnete Datenverbindung erfolgen.

Von den Reinigungsvorrichtungen 112 ist lediglich eine Reinigungsvorrichtung detaillierter, jedoch immer noch schematisch, dargestellt. Bei dieser Reinigungsvorrichtung 112 handelt es sich um einen Steckbeckenspüler 120, welcher zur Reinigung von Spülgut mit größerem Flüssigkeitsanfall (zum Beispiel Steckbecken, Nachttöpfe etc.) im Inneren einer Spülkammer 122 genutzt werden kann. Das Spülgut ist dabei in Figur 1 nicht dargestellt.

Der Steckbeckenspüler 120 kann beispielsweise ausgestaltet sein, wie aus dem Stand der Technik bekannt und wie beispielsweise in DE 103 48 344 A1 beschrieben. Dementsprechend weist der Steckbeckenspüler 120 neben der Spülkammer 122 eine Versorgungseinheit 124 auf, welche mit einem Wasserzulauf 126 verbunden ist und welche beispielsweise einen Wasserboiler und/oder einen Dampferzeuger umfassen kann (in Figur 1 nicht dargestellt). Die Versorgungseinheit 124 ist mit der Spülkammer 122 über Zuleitungen 128 verbunden, welche insbesondere in mehreren Düsen 130 oder Öffnungen im Inneren der Spülkammer 122 münden.

Die Spülkammer 122 weist an ihrem unteren Ende einen Ablauf 132 mit einem Flüssigkeitsvorrat gefüllten Siphonbogen 134 auf. Nach Einbringen des Spülgutes in die Spülkammer 122 und Schließen der Kammertüre 136 kann dann (beispielsweise automatisch durch eine Schwenkbewegung) das Spülgut in den Ablauf 132 entleert und somit von seinen Flüssigkeitsabfällen grob befreit werden. Anschließend kann über die Zuleitungen 128 eine Beaufschlagung mit kalter oder warmer Spülflüssigkeit (mit oder ohne Zusätze, wie beispielsweise Reinigungsmittel und/oder Desinfektionsmittel) erfolgen, und dadurch eine Grobspülung bzw. Grobreinigung bewirkt werden. Zur Erhöhung des Drucks der Spülflüssigkeit kann beispielsweise auch eine Pumpe 138 eingesetzt werden. Dadurch kann das Spülgut auch mit einem gewissen Düsendruck aus den Düsen 130 abgespritzt werden.

Bereits in diesem Grobreinigungsschritt kann eine gewisse desinfizierende Wirkung eintreten, beispielsweise indem Spülflüssigkeit mit einer erhöhten Temperatur (beispielsweise einer Temperatur von ca. 70 bis 80°C) verwendet wird, und/oder indem der Spülflüssigkeit Desinfektionsmittel beigemischt werden. Beispielsweise kann zu diesem Zweck die Versorgungseinheit 124 auch entsprechende Desinfektionsmitteltanks umfassen, wobei beispielsweise aus mehreren zur Verfügung stehenden Desinfektionsmitteln ein geeignetes Desinfektionsmittel ausgewählt werden kann oder wobei eine Desinfektionsmittelmenge einer zu erreichenden Desinfektionswirkung angepasst werden kann. Auch die Einwirkzeit des Desinfektionsmittels auf das Spülgut beeinflusst die Desinfektionswirkung. Die Einwirkzeit, die Temperatur und die Desinfektions- bzw. Spülmittelmenge und Art des Desinfektions- bzw. Spülmittels sind als Beispiele der steuerbaren Prozessparameter, die die Hygienewirkung beeinflussen und die, wie im Folgenden beschrieben, entsprechend einem oder mehreren vorgegebenen Zielkeimen ausgewählt werden können.

Anschließend an die beschriebene Flüssigkeitsreinigung kann, ebenfalls unter Zuhilfenahme der Versorgungseinheit 124, welche den Dampferzeuger beinhaltet, das Reinigungsgut auch mit Dampf beaufschlagt und dadurch weiter desinfiziert werden. Wiederum kann zu diesem Zweck das System der Zuleitung 128 und der Düsen 130 verwendet werden, um die Dampfdesinfektion durchzuführen. In diesem Fall bestimmen insbesondere die Dampftemperatur sowie die Einwirkzeit des Dampfes auf das Spülgut die Reinigungs- und Desinfektionswirkung, so dass diese Größen weitere Beispiele möglicher, die Hygienewirkung beeinflussender Prozessparameter darstellen, welche entsprechend dem bzw. den eingegebenen Zielkeimen ausgewählt werden können.

Anschließend an die Dampfdesinfektion kann der Dampf durch zwangsweises Einbringen von Zuluft durch eine Luftzuleitung 140 aus dem Inneren der Spülkammer 122 verdrängt werden und über eine Ableitung 142, welche über ein Rückschlagventil 144 verfügt, unter Umgehung des Siphonbogens 134 in den Ablauf 132 abgeleitet werden.

Der Reinigungsprozess des Steckbeckenspülers 120 kann durch die Steuerung 114 überwacht, gesteuert und/oder geregelt werden. Beispielsweise kann die Steuerung 114 auf die Funktionen der Versorgungseinheit 124 einwirken, um die entsprechenden Programmschritt nacheinander durchzuführen. Die Steuerung 114 kann beispielsweise eine in den Steckbeckenspüler 120 integrierte Steuerung sein, welche ein Display 146, Bedienelemente 148 sowie ein Computersystem mit einer entsprechenden Menüführung beinhaltet. Die Steuerung 114 kann über eine Schnittstelle 150 mit dem restlichen Steckbeckenspüler 120 Informationen oder Befehle austauschen.

Zur Überwachung der Reinigungswirkung können verschiedene Überwachungs- und Messvorrichtungen vorgesehen sein. Im vorliegenden Ausführungsbeispiel umfasst der Steckbeckenspüler 120 im Inneren der Spülkammer 122 an einer für die Beaufschlagung des Spülgutes repräsentativen Stelle mindestens einen Temperaturfühler 152, welcher über eine Leitung 154 Informationen an die Steuerung 114 liefert.

Es sei darauf hingewiesen, dass der in Figur 1 dargestellte Steckbeckenspüler 120 nur eine Möglichkeit aus einer Vielzahl von Reinigungsvorrichtungen 112 darstellt, bei welchen die Reinigungs- und Desinfektionswirkung bzw. die keimabtötende Wirkung durch einen oder mehrere Parameter beeinflusst werden kann. Im vorliegenden Fall wären dies beispielsweise die oben genannten Parameter wie Menge und/oder Art eines Desinfektionsmittels oder Reinigungsmittels, welches als Flüssigkeitszusatz der Spülflüssigkeit beigemischt wird, eine Einwirkzeit der derart zusammengesetzten Spülflüssigkeit, die Temperatur der Spülflüssigkeit, die Temperatur und/oder Dauer der Dampfdesinfektion oder ähnliche Parameter. Entsprechend können bei Geschirrspülmaschinen oder anderen Arten von Reinigungsvorrichtungen 112 andere Parameter relevant sein, wie beispielsweise eine Bandgeschwindigkeit, welche die Verweildauer des Spülgutes in einer Behandlungszone mit erhöhter Temperatur bestimmt.

In der Steuerung 114 oder an anderen Stellen der Reinigungsvorrichtung 112 bzw. des Steckbeckenspüler 120 können weiterhin noch Zeiterfassungsvorrichtungen vorgesehen sein, beispielsweise gewöhnliche Uhren und/oder Taktgeber eines Prozessors. Diese können beispielsweise genutzt werden, um unter gleichzeitiger Berücksichtigung der vom Temperaturfühler 152 gelieferten Signale Thermoäquivalente zu ermitteln, mit denen das Spülgut innerhalb der Spülkammer 122 beaufschlagt wurde.

Wie oben beschrieben, stellt das Reinigungssystem 110 gemäß dem Ausführungsbeispiel in Figur 1 ein dezentralisiertes Reinigungssystem dar, bei welchem die zentrale Steuerung 116 getrennt ist von den Steuerungen 114 der einzelnen Reinigungsvorrichtungen 112. Es ist jedoch genauso eine Ausführungsform denkbar, bei welcher das Reinigungssystem 110 lediglich eine einzelne Reinigungsvorrichtung 112 umfasst. In diesem Fall können beispielsweise auch die Steuerung 114 und die zentrale Steuerung 116 zu einer gemeinsamen Steuerung zusammengefasst sein. Auch ein Aufbau ist denkbar, bei welchem eine der Reinigungsvorrichtungen 112 als "Master" fungiert und andere Reinigungsvorrichtungen 112 ("Slaves") steuert. In diesem Fall kann beispielsweise die zentrale Steuerung 116 mit der Steuerung 114 des Masters zusammengefasst sein. Weitere Architekturen sind denkbar.

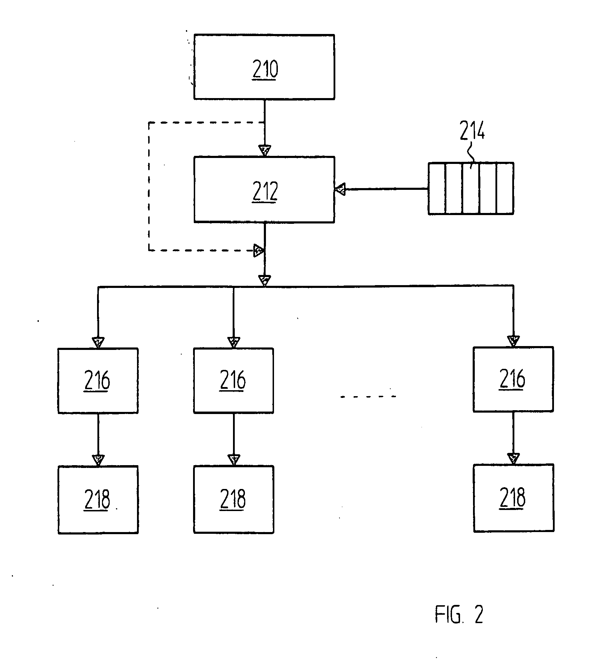

Anhand von Figur 2 soll im Folgenden ein möglicher Ablaufplan des erfindungsgemäßen Verfahrens beschrieben werden. Beispielsweise können die zentrale Steuerung 116 und/oder die Steuerung 114 der einzelnen Reinigungsvorrichtungen 112 im Figur 1 programmtechnisch eingerichtet sein, um das Verfahren durchzuführen.

In einem ersten Verfahrensschritt, welcher in Figur 2 mit 210 bezeichnet ist, wird eine Information über einen Zielkeim empfangen. Beispielsweise kann die zentrale Steuerung 116 zu diesem Zweck wiederum Bedienelemente 156 und ein Display 158 umfassen, so dass beispielsweise ein Benutzer aus einer Datenbank oder Liste (beispielsweise menügesteuert) einen oder mehrere Zielkeime auswählen kann. Dies kann beispielsweise durch eine medizinisch/technische Assistentin (MTA) in einem Krankenhaus oder durch Pflegepersonal in einem Pflegeheim erfolgen. Auch eine zentrale Steuerung, beispielsweise über einen von einem Hygieniker bedienten Zentralrechner als zentrale Steuereinheit 116 in einem Krankenhaus oder eine als Arztterminal ausgestaltete zentrale Steuereinheit 116 ist denkbar.

Alternativ oder zusätzlich kann die zentrale Steuereinheit 116 auch über eine drahtgebundene oder drahtlose Datenfernübertragungseinrichtung 160 verfügen, beispielsweise einen Anschluss an das Internet oder ein größeres Computernetzwerk. Auch hierüber können Informationen über die abzutötenden Zielkeime empfangen werden.

Anschließend wird in Verfahrensschritt 212 in dem in Figur 2 dargestellten Ausführungsbeispiel mit der zentralisierten Steuerung die Information über den Zielkeim in ein entsprechendes Zielthermoäquivalent umgewandelt. Hierzu werden entsprechende Zielthermoäquivalente eingelesen, was in Figur 2 symbolisch mit der Bezugsziffer 214 bezeichnet ist. Beispielsweise kann dies einen Vergleich von eingegebenen Zielkeimen mit einer Tabelle beinhalten, wie oben beschrieben. Beispielsweise kann für diesen Zweck eine elektronische Tabelle (z.B. eine Lookup-Table) oder eine Datenbank vorgesehen sein.

Werden anstelle von Zielthermoäquivalenten unmittelbar Informationen über die Zielkeime an die einzelnen Reinigungsvorrichtungen 112 weitergeleitet, so kann der Verfahrensschritt 212, wie in Figur 2 durch die gestrichelte Linie dargestellt, auch umgangen werden. Beide Verfahrensvarianten sind möglich, oder auch Kombinationen der beschriebenen Verfahrensvarianten, bei welchem an die einzelnen Reinigungsvorrichtungen 112 des Reinigungssystems 110 sowohl Zielthermoäquivalente als auch "rohe" Informationen über die zu erwartenden Zielkeime weitergeleitet werden. In letzterem Fall kann beispielsweise in den einzelnen Reinigungsvorrichtungen 112 ein kombiniertes Reinigungsprogramm durchgeführt werden, bei welchem einerseits die Einhaltung vorgegebener Zielthermoäquivalente überwacht werden kann und bei welchem andererseits auch andere Parameter speziell auf den Zielkeim abgestimmt werden können, beispielsweise die Art und/oder die Dosierung und/oder die Einwirkzeit eines Desinfektionsmittels oder ähnliches.

Die Zielthermoäquivalente bzw. die Informationen über die Zielkeime werden dann an die einzelnen Reinigungsvorrichtungen 112 weitergeleitet. In diesen einzelnen Reinigungsvorrichtungen 112 werden entsprechend dieser Vorgaben dann geeignete Prozessparameter ausgewählt, was in Figur 2 mit der Bezugsziffer 216 bezeichnet ist. Diese Auswahl kann beispielsweise in den Steuerungen 114 der Reinigungsvorrichtungen 112 erfolgen. Beispielsweise kann wiederum die von der zentralen Steuerung 116 empfangene Information (Information über den Zielkeim und/oder ein Zielthermoäquivalent) durch Vergleich mit einer Liste, einer Tabelle oder einer Datenbank in geeignete Prozessparameter umgesetzt werden. Die Prozessparameter sind derart gewählt, dass diese den in Verfahrensschritt 210 benannten Zielkeim mit hoher Wahrscheinlichkeit abtöten.

Im Falle der Vorgabe von Zielthermoäquivalenten kann dies beispielsweise dadurch geschehen, dass als Prozessparameter eine Temperatur in der Spülkammer 122 während eines bestimmten Verfahrensschrittes vorgegeben wird, sowie eine bestimmte Verweildauer innerhalb dieser Temperatur. Auch ein einfaches Akkumulieren von Thermoäquivalenten ist möglich, wobei dann beispielsweise der Reinigungsprozess abgebrochen wird, sobald das entsprechende Zielthermoäquivalent erreicht ist.

Anschließend wird in Verfahrensschritt 218 der Reinigungsprozess mit den in Verfahrensschritt 216 jeweils ausgewählten Prozessparametern durchgeführt. Wie oben beschrieben, kann dieser Reinigungsprozess auch mehrstufig ausgebildet sein, kann beispielsweise mehrere Programmschritte umfassen, so dass insbesondere auch mehrere Prozessparametersätze für die einzelnen Verfahrensschritte verwendet werden können.

### Bezugszeichenliste

- 110: Reinigungssystem
- 112: Reinigungsvorrichtungen
- 114: Steuerung
- 116: zentrale Steuerung
- 118: Datenverbindung
- 120: Steckbeckenspüler
- 122: Spülkammer
- 124: Versorgungseinheit
- 126: Wasserzulauf
- 128: Zuleitungen
- 130: Düsen
- 132: Ablauf
- 134: Siphonbogen
- 136: Kammertür
- 138: Pumpe
- 140: Luftzuleitung
- 142: Ableitung
- 144: Rückschlagventil
- 146: Display
- 148: Bedienelemente
- 150: Schnittstelle
- 152: Temperaturfühler
- 154: Leitung
- 156: Bedienelemente
- 158: Display
- 160: Datenfernübertragungseinrichtung

- 210: empfangene Information Zielkeim
- 212: Umwandlung Zielthermoäquivalent
- 214: Einlesen Zielthermoäquivalent
- 216: Auswahl Prozessparameter
- 218: Durchführung Reinigungsprozess

## Patentansprüche

1. Verfahren zum Betrieb eines eine Reinigungsvorrichtung (112) umfassenden Reinigungssystems (110), wobei die Reinigungsvorrichtung (112) eingerichtet ist, um einen Reinigungsprozess eines Objektes mit keimtötender Wirkung durchzuführen, wobei das Verfahren folgende Schritte umfasst:
a) eine Information über einen Zielkeim, mit dem das Objekt kontaminiert sein könnte, wird von dem Reinigungssystem (110) empfangen;
b) entsprechend dem Zielkeim wird mindestens ein Prozessparameter des Reinigungsprozesses von dem Reinigungssystem (110) derart gewählt, dass der Zielkeim bei dem Reinigungsprozess mit hoher Wahrscheinlichkeit abgetötet wird, wobei die Wahl des Prozessparameters durch das Reinigungssystem (110) erfolgt,
wobei dem Zielkeim ein Erfahrungswert eines entsprechenden Zielthermoäquivalents zugeordnet wird, bei welchem der Zielkeim mit hoher Wahrscheinlichkeit abgetötet ist, wobei der Reinigungsprozess derart durchgeführt wird, dass das Objekt mindestens mit dem Zielthermoäquivalent beaufschlagt wird; und
c) der Reinigungsprozess wird mit dem Prozessparameter durchgeführt.

2. Verfahren nach dem vorhergehenden Anspruch, wobei der mindestens eine Prozessparameter mindestens einen der folgenden Parameter umfasst: eine Temperatur; eine Einwirkzeit eines Reinigungsschrittes mit einer Reinigungsflüssigkeit; eine Temperatur einer Reinigungsflüssigkeit; eine Temperatur eines Temperaturbehandlungsschrittes; eine Einwirkzeit eines Temperaturbehandlungsschrittes; eine Auswahl eines Desinfektionsmittels; eine Art eines Desinfektionsmittels; eine Menge eines Desinfektionsmittels; eine Einwirkzeit eines Desinfektionsmittels; eine Dampftemperatur eines Dampfdesinfektionsschrittes; eine Einwirkzeit eines Dampfdesinfektionsschrittes.

3. Verfahren nach einem der vorhergehenden Ansprüche, wobei das Zielthermoäquivalent einem Thermoäquivalent nach dem EN ISO 15883-Standard oder dem NSF3-Standard entspricht.

4. Verfahren nach dem vorhergehenden Anspruch, wobei das Zielthermoäquivalent einen A₀-Wert und/oder einen H.U.E.-Wert umfasst.

5. Verfahren nach einem der vorhergehenden Ansprüche, wobei bei dem Reinigungsprozess eine Temperatur gemessen wird, mit der das Objekt beaufschlagt wird, und eine Einwirkzeit, in der das Objekt mit der Temperatur beaufschlagt wird.

6. Verfahren nach dem vorhergehenden Anspruch, wobei die Temperatur und/oder die Einwirkzeit geregelt oder gesteuert werden.

7. Verfahren nach einem der vorhergehenden Ansprüche, wobei in Verfahrensschritt b) eine Zuordnung eines oder mehrerer Zielkeime zu einem oder mehreren Prozessparametern verwendet wird, wobei die Zuordnung mindestens eines der folgenden Elemente umfasst: eine Datenbank; eine Liste; eine Tabelle; eine Matrix; eine Korrelationskurve.

8. Verfahren nach dem vorhergehenden Anspruch, wobei mehrere Zielkeime zu Zielkeimgruppen oder Zielkeimklassen mit gemeinsamen Parametern zusammengefasst sind.

9. Verfahren nach einem der vorhergehenden Ansprüche, wobei der Zielkeim in Verfahrensschritt a) entsprechend einer regionalen, nationalen oder globalen Häufigkeit oder Häufigkeitsverteilung von Infektionen und/oder einer Seuchenwamung und/oder einer medizinischen Diagnose ausgewählt wird.

10. Verfahren nach einem der vorhergehenden Ansprüche, wobei der Reinigungsprozess, unabhängig von dem Zielerreger, mindestens mit einem Mindestparametersatz zur Erreichung einer keimtötenden Mindestwirkung durchgeführt wird.

11. Reinigungssystem (110) zur Reinigung eines Objektes, insbesondere unter Verwendung eines Verfahrens nach einem der vorhergehenden Ansprüche, wobei das Reinigungssystem (110) eine Reinigungsvorrichtung (112) umfasst, welche eingerichtet ist, um einen Reinigungsprozess des Objektes mit keimtötender Wirkung durchzuführen,
a) wobei das Reinigungssystem (110) eingerichtet ist, um eine Information über einen Zielkeim zu empfangen, mit dem das Objekt kontaminiert sein könnte;
b) wobei das Reinigungssystem (110) eingerichtet ist, um entsprechend dem Zielkeim mindestens einen Prozessparameter des Reinigungsprozesses derart zu wählen, dass der Zielkeim bei dem Reinigungsprozess mit hoher Wahrscheinlichkeit abgetötet wird, wobei dem Zielkeim ein Erfahrungswert eines entsprechenden Zielthermoäquivalents zugeordnet wird, bei welchem der Zielkeim mit hoher Wahrscheinlichkeit abgetötet ist, wobei der Reinigungsprozess derart durchgeführt wird, dass das Objekt mindestens mit dem Zielthermoäquivalent beaufschlagt wird; und
c) wobei das Reinigungssystem (110) eingerichtet ist, um die Reinigungsvorrichtung (112) derart zu steuern, dass der Reinigungsprozess mit dem Prozessparameter durchgeführt wird.

12. Reinigungssystem (110) nach dem vorhergehenden Anspruch, wobei das Reinigungssystem (110) eine Eingabevorrichtung (148, 156) umfasst, die einem Benutzer die Eingabe eines Zielkeims oder einer Zielkeimklasse, insbesondere die Auswahl eines Zielkeims oder einer Zielkeimklasse aus einer Zielkeimliste oder Zielkeimklassenliste, ermöglicht.

13. Reinigungssystem (110) nach einem der beiden vorhergehenden Ansprüche, wobei das Reinigungssystem (110) eine Schnittstelle (160) umfasst, um Informationen über eine regionale, nationale oder globale Häufigkeit oder Häufigkeitsverteilung von Infektionen und/oder eine Seuchenwarnung und/oder Informationen aus einer medizinischen Patientendatenbank zu empfangen.

14. Reinigungssystem (110) nach einem der drei vorhergehenden Ansprüche, wobei die Reinigungsvorrichtung (112) mindestens eine der folgenden Reinigungsvorrichtungen (112) umfasst: eine Geschirrspülmaschine, insbesondere eine gewerbliche Geschirrspülmaschine, insbesondere eine Einkammergeschirrspülmaschine oder eine Durchlaufgeschirrspülmaschine; eine Reinigungsvorrichtung (112) für die Reinigung von medizinischen Geräten oder Pflegeutensilien mit hohem Flüssigkeitsanfall, insbesondere einen Steckbeckenspüler (120); eine Reinigungsvorrichtung (112) für die Desinfektion von medizinischen Geräten ohne oder mit geringem Flüssigkeitsanfall, insbesondere eine thermische Desinfektionsvorrichtung, insbesondere einen Autoklaven, und/oder eine Dampfdesinfektionsvorrichtung.

15. Reinigungssystem (110) nach einem der vorhergehenden, auf ein Reinigungssystem (110) gerichteten Ansprüchen, wobei das Reinigungssystem (110) eine Mehrzahl von Reinigungsvorrichtungen (112) und eine zentrale Steuerung (116) umfasst, wobei die zentrale Steuerung (116) eingerichtet ist, um zentral für die Reinigungsvorrichtungen (112) die Information über den Zielkeim zu empfangen.

16. Reinigungssystem (110) nach dem vorhergehenden Anspruch, wobei die zentrale Steuerung (116) eingerichtet ist, um dem Zielkeim ein Zielthermoäquivalent zuzuordnen und eine Information über das Zielthermoäquivalent an die Reinigungsvorrichtungen (112) weiterzuleiten, wobei die Reinigungsvorrichtungen (112) eingerichtet sind, um jeweils einen Reinigungsprozess derart durchzuführen, dass die zu reinigenden Objekte mit dem Zielthermoäquivalent beaufschlagt werden.

## Claims

1. Method for operating a cleaning system (110) comprising a cleaning device (112), the cleaning device (112) being set up for carrying out a process of cleaning an article with a germicidal action, the method comprising the following steps:
a) information on a target germ with which the article could be contaminated is received by the cleaning system (110);
b) at least one process parameter of the cleaning process is selected by the cleaning system (110) according to the target germ in such a way that the target germ is killed with a high degree of probability during the cleaning process, the selection of the process parameter being carried out by the cleaning system (110), the target germ being assigned an experimental value of a corresponding target thermal equivalent, at which the target germ is killed with a high degree of probability, the cleaning process being carried out in such a way that the article is acted upon at least with the target thermal equivalent; and
c) the cleaning process is carried out with the process parameter.

2. Method according to the preceding claim, the at least one process parameter comprising at least one of the following parameters: a temperature; a time of action of a cleaning step with a cleaning liquid; a temperature of a cleaning liquid; a temperature of a thermal treatment step; a time of action of a thermal treatment step; a selection of a disinfectant; a type of a disinfectant; a quantity of a disinfectant; a time of action of a disinfectant; a steam temperature of a steam disinfection step; a time of action of a steam disinfection step.

3. Method according to one of the preceding claims, the target thermal equivalent corresponding to a thermal equivalent according to the EN ISO 15883 standard or to the NSF3 standard.

4. Method according to the preceding claim, the target thermal equivalent comprising an A₀ value and/or an H.U.E. value.

5. Method according to one of the preceding claims, a temperature with which the article is acted upon and a time of action in which the article is acted upon with the temperature being measured during the cleaning process.

6. Method according to the preceding claim, the temperature and/or the time of action being regulated or controlled.

7. Method according to one of the preceding claims, in method step b) an assignment of one or more target germs to one or more process parameters being used, the assignment comprising at least one of the following elements: a database; a list; a table; a matrix; a correlation curve.

8. Method according to the preceding claim, a plurality of target germs being combined into target germ groups or target germ classes with common parameters.

9. Method according to one of the preceding claims, in method step a) the target germ being selected according to a regional, national or worldwide frequency or frequency distribution of infections and/or epidemic warning and/or a medical diagnosis.

10. Method according to one of the preceding claims, the cleaning process being carried out, independently of the target pathogen, at least with a minimum parameter set for achieving a minimum germicidal action.

11. Cleaning system (110) for cleaning an article, in particular using a method according to one of the preceding claims, the cleaning system (110) comprising a cleaning device (112) which is set up for carrying out a process of cleaning the article with a germicidal action,
a) the cleaning system (110) being set up for receiving information on a target germ with which the article could be contaminated;
b) the cleaning system (110) being set up for selecting at least one process parameter of the cleaning process according to the target germ in such a way that the target germ is killed with a high degree of probability during the cleaning process, the target germ being assigned an experimental value of a corresponding target thermal equivalent, at which the target germ is killed with a high degree of probability, the cleaning process being carried out in such a way that the article is acted upon at least with the target thermal equivalent; and
c) the cleaning system (110) being set up for controlling the cleaning device (112) in such a way that the cleaning process is carried out with the process parameter.

12. Cleaning system (110) according to the preceding claim, the cleaning system (110) comprising an input device (148, 156) which enables a user to enter a target germ or a target germ class, in particular to select a target germ or a target germ class from a target germ list or target germ class list.

13. Cleaning system (110) according to one of the two preceding claims, the cleaning system (110) comprising an interface (160) in order to receive information on a regional, national or worldwide frequency or frequency distribution of infections and/or an epidemic warning and/or information from a medical patient database.

14. Cleaning system (110) according to one of the three preceding claims, the cleaning device (112) comprising at least one of the following cleaning devices (112): a dishwasher, in particular an industrial dishwasher, in particular a single-chamber dishwasher or a continuous-flow dishwasher; a cleaning device (112) for cleaning medical implements or nursing utensils with a high incidence of liquids, in particular a basin washer (120); a cleaning device (112) for the disinfection of medical instruments without or with a low incidence of liquids, in particular a thermal disinfection device, in particular an autoclave, and/or a steam disinfection device.

15. Cleaning system (110) according to one of the preceding claims directed at a cleaning system (110), the cleaning system (110) comprising a plurality of cleaning devices (112) and a central control (116), the central control (116) being set up for receiving the information on the target germ centrally for the cleaning devices (112).

16. Cleaning system (110) according to the preceding claim, the central control (116) being set up for assigning a target thermal equivalent to the target germ and for transferring information on the target thermal equivalent to the cleaning devices (112), the cleaning devices (112) being set up for carrying out in each case a cleaning process in such a way that the articles to be cleaned are acted upon with the target thermal equivalent.

## Revendications

1. Procédé pour faire fonctionner un système de nettoyage (110) comprenant un dispositif de nettoyage (112), le dispositif de nettoyage (112) étant réalisé pour effectuer un processus de nettoyage d'un objet avec effet germicide, le procédé comportant les étapes suivantes :
a) une information sur le germe cible, avec lequel l'objet pourrait être contaminé, est reçue par le système de nettoyage (110) ;
b) au moins un paramètre de processus du processus de nettoyage est sélectionné par le système de nettoyage (110) en fonction du germe cible de telle sorte que le germe cible est très probablement détruit lors du processus de nettoyage, le paramètre de processus étant sélectionné par le système de nettoyage (110), une valeur empirique d'un équivalent thermique cible correspondant, pour laquelle le germe cible est très probablement détruit, étant affectée au germe cible, le processus de nettoyage étant réalisé de telle sorte que l'objet est au moins soumis à l'équivalent thermique cible ; et
c) le processus de nettoyage est réalisé avec le paramètre de processus.

2. Procédé selon la revendication précédente, le au moins un paramètre de processus comprenant au moins un des paramètres suivants : une température ; un temps d'action d'une étape de nettoyage avec un fluide de nettoyage ; une température d'un fluide de nettoyage ; une température d'une étape de traitement thermique ; un temps d'action d'une étape de traitement thermique ; une sélection d'un désinfectant ; un type de désinfectant ; une quantité de désinfectant ; un temps d'action d'un désinfectant ; une température de la vapeur d'une étape de désinfection à la vapeur ; un temps d'action d'une étape de désinfection à la vapeur.

3. Procédé selon l'une quelconque des revendications précédentes, l'équivalent thermique cible correspondant à un équivalent thermique selon la norme EN ISO 15883 ou la norme NSF3.

4. Procédé selon la revendication précédente, l'équivalent thermique cible comprenant une valeur A₀ et/ou une valeur H.U.E.

5. Procédé selon l'une quelconque des revendications précédentes, une température, à laquelle l'objet est soumis, et un temps d'action, pendant lequel l'objet est soumis à la température, étant mesurés lors du processus de nettoyage.

6. Procédé selon la revendication précédente, la température et/ou le temps d'action étant régulés ou commandés.

7. Procédé selon l'une quelconque des revendications précédentes, dans lequel à l'étape de procédé b) une affectation d'un ou de plusieurs germes cibles à un ou plusieurs paramètres de processus est utilisée, l'affectation comprenant au moins un des éléments suivants : une base de données ; une liste, un tableau ; une matrice ; une courbe de corrélation.

8. Procédé selon la revendication précédente, plusieurs germes cibles étant regroupés en groupes de germes cibles ou en classes de germes cibles avec des paramètres communs.

9. Procédé selon l'une quelconque des revendications précédentes, le germe cible étant sélectionné à l'étape de procédé a) en fonction d'une fréquence ou d'une distribution de fréquence régionale, nationale ou mondiale d'infections et/ou d'une alerte épidémie et/ou d'un diagnostic médical.

10. Procédé selon l'une quelconque des revendications précédentes, le processus de nettoyage étant réalisé, indépendamment de l'agent pathogène cible, avec au moins un jeu de paramètres minimum pour obtenir un effet germicide minimum.

11. Système de nettoyage (110) pour le nettoyage d'un objet, utilisant en particulier un procédé selon l'une quelconque des revendications précédentes, le système de nettoyage (110) comprenant un dispositif de nettoyage (112), qui est réalisé pour effectuer un processus de nettoyage de l'objet avec effet germicide,
a) le système de nettoyage (110) étant réalisé pour recevoir une information sur un germe cible avec lequel l'objet pourrait être contaminé ;
b) le système de nettoyage (110) étant réalisé pour sélectionner au moins un paramètre de processus du processus de nettoyage en fonction du germe cible de telle sorte que le germe cible est très probablement détruit lors du processus de nettoyage, une valeur empirique d'un équivalent thermique cible correspondant, pour laquelle le germe cible est très probablement détruit, étant affectée au germe cible, le processus de nettoyage étant réalisé de telle sorte que l'objet est au moins soumis à l'équivalent thermique cible ; et
c) le système de nettoyage (110) étant réalisé pour commander le dispositif de nettoyage (112) de telle sorte que le processus de nettoyage est réalisé avec le paramètre de processus.

12. Système de nettoyage (110) selon la revendication précédente, le système de nettoyage (110) comprenant un dispositif de saisie (148, 156), qui permet à un utilisateur de saisir un germe cible ou une classe de germes cibles, en particulier de sélectionner un germe cible ou une classe de germes cibles à partir d'une liste de germes cibles ou d'une liste de classes de germes cibles.

13. Système de nettoyage (110) selon l'une quelconque des deux revendications précédentes, le système de nettoyage (110) comprenant une interface (160) pour recevoir des informations sur une fréquence ou distribution de fréquence régionale, nationale ou mondiale d'infections et/ou une alerte épidémie et/ou des informations provenant d'une base de données médicale de patients.

14. Système de nettoyage (110) selon l'une quelconque des trois revendications précédentes, le dispositif de nettoyage (112) comprenant au moins un des dispositifs de nettoyage (112) suivants : un lave-vaisselle, en particulier un lave-vaisselle industriel, plus particulièrement un lave-vaisselle à une cuve ou un lave-vaisselle à passage ; un dispositif de nettoyage (112) pour le nettoyage d'appareils médicaux ou d'ustensiles de soins avec une grande production de liquide, en particulier un lave-bassins (120) ; un dispositif de nettoyage (112) pour la désinfection d'appareils médicaux sans stockage de liquide ou avec un faible stockage de liquide, en particulier un dispositif de désinfection thermique, en particulier un autoclave et/ou un dispositif de désinfection à la vapeur.

15. Système de nettoyage (110) selon l'une quelconque des revendications précédentes portant sur un système de nettoyage (110), le système de nettoyage (110) comprenant une pluralité de dispositifs de nettoyage (112) et une commande centrale (116), la commande centrale (116) étant réalisée pour recevoir l'information sur le germe cible de manière centralisée pour les dispositifs de nettoyage (112).

16. Système de nettoyage (110) selon la revendication précédente, la commande centrale (116) étant réalisée pour affecter un équivalent thermique cible au germe cible et pour transmettre une information sur l'équivalent thermique cible aux dispositifs de nettoyage (112), les dispositifs de nettoyage (112) étant réalisés pour effectuer respectivement un processus de nettoyage de telle sorte que les objets à nettoyer sont soumis à l'équivalent thermique cible.
